# EUROPÄISCHE PATENTSCHRIFT

(11) **EP 0 878 178 B1**
(45) Veröffentlichungstag und Bekanntmachung des Hinweises auf die Patenterteilung: **05.03.2003**
(21) Anmeldenummer: 98105245.9
(22) Anmeldetag: 23.03.1998
(51) Int. Cl.: A61F 2/52

(54) **Brustprothese**
Mammary prosthesis
Prothèse mammaire

(30) Priorität: 15.05.1997 DE 29708685 U; 24.07.1997 DE 29713203 U
(43) Veröffentlichungstag der Anmeldung: 18.11.1998
(73) Patentinhaber: AMOENA Medizin-Orthopädie-Technik GmbH, D-83064 Raubling (DE)
(72) Erfinder: Reitmaier, Paul, 83547 Babensham (DE)
(74) Vertreter: Laufhütte, Dieter, Dr.-Ing.

(56) Entgegenhaltungen:
- DE-U- 9 306 572
- DE-U- 9 315 935
- DE-U- 29 607 969
- DE-U- 29 713 203

## Beschreibung

Die Erfindung betrifft eine Brustprothese, die ein erstes elastisches Material, vorzugsweise eine weich-elastisch eingestellte additionsvernetzende Zwei-Komponenten-Silikon-Kautschuk-Masse sowie einen mit der Brustprothese verbindbaren Tragestreifen zum Zwecke der Befestigung der Brustprothese an der Brust der Trägerin umfaßt.

Eine derartige Brustprothese ist aus dem DE-GM 91 14 512 bekannt. Hier wird zur Befestigung der Brustprothese an der Brust der Trägerin eine mit einem Klebemittel versehene und als Tragestreifen dienende Schicht vorgesehen, die sich im Randbereich der Brustprothese erstreckt und über ihre gesamte Breite mit der Brustprothese verbunden ist. Trotz einer guten Klebefähigkeit des Klebemittels ist aufgrund möglicher ungünstiger Belastungen während der Bewegung eine sichere Verbindung der Brustprothese mit der Brust der Trägerin nicht immer gewährleistet. Die ungünstigen Belastungen beruhen im wesentlichen darauf, daß durch ein mögliches Kippen der Brustprothese und somit der Klebeschicht derart hohe Belastungen in den Randbereichen der Klebeschicht auftreten, daß ein Ablösen der Brustprothese von der Brust nicht verhindert werden kann.

In dem DE-GM 93 06 572 wird eine Brustprothese offenbart, mit der dieser Nachteil dadurch behoben wird, daß der Tragestreifen nur in einem mittleren Bereich mit einer Klebeschicht versehen ist, so daß eine Verbindung des Tragestreifens mit der Brustprothese entsprechend nur in diesem Bereich vorliegt. Dadurch wird erreicht, daß auch bei kippender Bewegung der Brustprothese nur der mittlere Bereich belastet wird, so daß auf die Ränder des Tragestreifens keine Kräfte eingeleitet werden und somit ein Ablösen des Tragestreifens von der Brust der Trägerin vermieden werden kann. Eine derartige Ausführung einer Brustprothese führt zu dem gewünschten Erfolg einer günstigen Krafteinleitung, jedoch kann beim Abnehmen der Brustprothese das Problem auftreten, daß es aufgrund der nur mittigen Verbindung des Tragestreifens mit der Brustprothese verhältnismäßig leicht zu einer Beschädigung des Tragestreifens bzw. der Brustprothese kommen kann. Die fehlende Fixierung der Randbereiche des Tragestreifens erschwert wegen der sich bildenden Spalte ferner eine Reinigung in diesem Bereich und kann darüber hinaus zu einem Verkleben der Randbereiche des Tragestreifens während und nach dem Abnehmen der Brustprothese führen.

In der DE 296 07 969 U1 ist eine Brustprothese gemäß dem Oberbegriff von Anspruch 1 der gleichen Anmelderin beschrieben, die aus zwei jeweils in Kunststoffolien eingeschweißten schalenförmigen Körpern aus Silikonmasse unterschiedlicher Weichheit besteht, wobei der äußere der Brustform nachgebildete Körper eine Härte besitzt, die der weich-elastischen Nachgiebigkeit des natürlichen Brustgewebes angeglichen ist, und der innere Körper eine weichere, gelartige Konsistenz aufweist. Auf der Rückseite dieser Prothese ist in ihrem Randbereich eine dauernd klebrig bleibende Haftschicht vorgesehen, wobei ein erster Bereich der Haftschicht auf dem äußeren Prothesenkörper und ein zweiter Bereich der Haftschicht auf dem inneren Körper angeordnet ist.

Aufgabe der vorliegenden Erfindung ist es, eine gattungsgemäße Brustprothese zu schaffen, die eine sichere und dauerhafte Befestigung gewährleistet und ein problemloses und beschädigungsfreies Abnehmen ermöglicht.

Diese Aufgabe wird erfindungsgemäß durch die in Anspruch 1 definierte Brustprothese gelöst. Durch diese Art der Verbindung wird bei Bewegungen der Brustprothese nur der mittlere, mit dem ersten elastischen Material der Brustprothese verbundene Bereich des Tragestreifens belastet. Während der auftretenden kippenden oder schwenkenden Bewegungen der Brustprothese im Bereich des Tragestreifens wird die Kraft auf die Randbereiche des Tragestreifens dadurch verringert, daß diese das zweite elastische Material, das erfindungsgemäß eine höhere Elastizität als das erste elastische Material der Brustprothese aufweist, mit entsprechend geringerem Kraftaufwand elastisch verformen. Der geringe Kraftaufwand führt zu einer geringen Krafteinleitung in die Randbereiche des Tragestreifens. Durch die Verwendung des zweiten elastischen Materials ist es somit möglich, daß die Brustprothese im Bereich des Tragestreifens aufgrund der hohen Elastizität des zweiten elastischen Materials bewegt wird, ohne daß dabei im Randbereich des Tragestreifens das Ablösen des Tragestreifens von der Brust der Trägerin bewirkende Kräfte eingeleitet werden. Der mittlere an dem ersten elastischen Material der Brustprothese befestigte Bereich des Tragestreifens wirkt somit gleichsam als Scharnier während die Randbereiche des Tragestreifens je nach Art der Belastung geringfügig in das zweite elastische Material hereingerückt werden oder dieses entsprechend nach außen verformen.

Besonders vorteilhaft ist es, wenn das zweite elastische Material in Form zweier Streifen ausgebildet ist, die sich auf beiden Randbereichen des Tragestreifens erstrecken. Dabei können die Streifen bereits während der Verarbeitung bzw. Formgebung des ersten elastischen Materials mit in die Brustprothese eingebracht werden oder auch nachträglich in entsprechende Aussparungen des ersten elastischen Materials eingelegt und dort befestigt werden.

In einer bevorzugten Ausgestaltung der Erfindung ist vorgesehen, daß eine oder mehrere der an die Brustprothese angrenzenden Seiten der Streifen eine gekrümmte Oberfläche aufweisen. Dabei können die Streifen beispielsweise die Querschnittsform eines Kreissegmentes aufweisen, wodurch ungünstige Krafteinleitungen durch Ecken und Kanten vermieden werden.

Besonders vorteilhaft ist es, wenn sich das zweite elastische Material der Brustprothese von einem oder beiden der Randbereiche des Tragestreifens in weitere Bereiche der Brustprothese erstreckt. Dadurch lassen sich bei der Verwendung von Zwei-Schicht-Prothesen die günstigen Eigenschaften eines mehrere Komponenten umfassenden Aufbaus der Brustprothese mit der erfindungsgemäßen Ausgestaltung der Randbereiche eines Tragestreifens verbinden.

Gemäß einer bevorzugten Ausgestaltung der vorliegenden Erfindung ist vorgesehen, daß der Tragestreifen in einem Randbereich der Brustprothese angeordnet ist. Dadurch wird zum einen erreicht, daß sich die auftretenden Belastungen durch das Gewicht der Brustprothese sowie die auftretenden Bewegungen gleichmäßig über die Randbereiche der Brustprothese verteilen. Zum anderen wird durch die Anordnung des Tragestreifens im Randbereich der Brustprothese eine entsprechend größere Verbindungsfläche des Tragestreifens mit der Brustprothese sowie mit der Brust der Trägerin und somit eine stabilere Verbindung erzielt. Insbesondere ergibt sich ein gleichmäßiger Übergang von der Prothese zum Körper der Trägerin. Wird der Tragestreifen nicht im Randbereich der Brustprothese, sondern in einem davon beabstandeten Bereich vorgesehen, kann sich in Abhängigkeit der Bewegungen der Trägerin die Außenkante der Prothese unter Umständen vom Körper abheben.

Die Verbindung zwischen dem Tragestreifen und der Brustprothese kann lösbar ausgeführt sein, was insbesondere dann von Vorteil ist, wenn sich beispielsweise Beschädigungen des Tragestreifens oder eine Unverträglichkeit mit der Haut ergeben haben. Die Fixierung des Tragestreifens an der Brustprothese kann dabei mittels einer lösbaren Klebeschicht oder auch einer Klettverbindung erreicht werden.

In weiterer Ausgestaltung der Erfindung ist vorgesehen, daß der Tragestreifen mit einer dauernd klebrigen Schicht versehen ist, wobei diese auf einer oder auf beiden Seiten des Tragestreifens vorhanden sein kann.

Die Verbindung des Tragestreifens mit der Brustprothese kann in voneinander beabstandeten Bereichen erfolgen. Durch eine derartige abschnittsweise Verbindung wird gegenüber einer durchgehenden Fixierung des Tragestreifens eine Steigerung der Beweglichkeit und Flexibilität bei unterschiedlichen Belastungen erreicht.

Besonders vorteilhaft ist es, wenn der Tragestreifen auf seiner zu der Brustprothese zugewandten Seite abgerundete Kanten aufweist. Dadurch wird trotz des ständigen Kontaktes der Randbereiche des Tragestreifens mit der Brustprothese eine Beschädigung des zweiten elastischen Materials durch Ecken oder Kanten weitgehend vermieden.

Weitere Einzelheiten und Vorteile der Erfindung werden anhand eines in der Zeichnung dargestellten Ausführungsbeispiels näher erläutert. Es zeigen:
- Fig. 1:: einen Querschnitt durch die erfindungsgemäße Brustprothese mit Tragestreifen und als Streifen ausgeführtem zweiten elastischen Material im Randbereich des Tragestreifens und
- Fig. 2:: einen Querschnitt durch eine erfindungsgemäße Zwei-Komponenten-Brustprothese mit Tragestreifen.

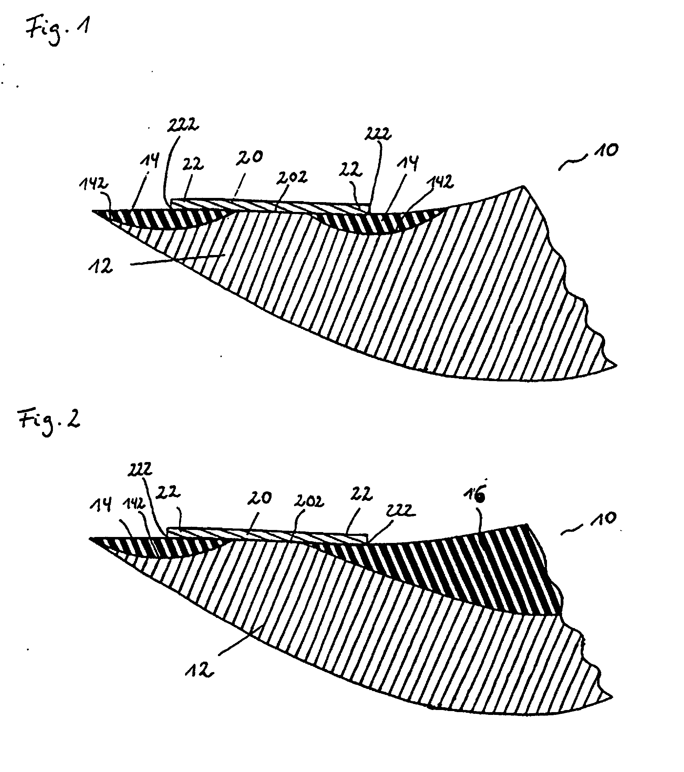

Fig. 1 zeigt eine erfindungsgemäße Brustprothese 10, die ein erstes elastisches Material 12 umfaßt. Im Randbereich der Brustprothese 10 befinden sich ein zweites elastisches Material 14 aufweisende Streifen. Ein Tragestreifen 20 der Brustprothese 10 ist derart angeordnet, daß sich seine Randbereiche 22 in den Bereich des zweiten elastischen Materials 14 erstrecken. Durch eine Bewegung der Brustprothese 10 wird je nach der Art der auftretenden Belastung beispielsweise einer der Randbereiche 22 des Tragestreifens 20 in das zweite elastische Material 14 eingedrückt, während der andere der Randbereiche 22 des Tragestreifens 20 eine entsprechende Dehnung des zweiten elastischen Materials 14 bewirkt. Dabei bewegt sich die Brustprothese 10 aufgrund der höheren Elastizität des zweiten elastischen Materials 14 kippend um den an dem ersten elastischen Material 12 befestigten mittleren Bereich des Tragestreifens 20. Durch die erfindungsgemäß höhere Elastizität des zweiten elastischen Materials 14 können auf diese Weise unerwünschte Belastungen und Krafteinleitungen, die zu einem Ablösen des Tragestreifens 20 und somit der Brustprothese 10 von der Brust der Trägerin führen könnten, vermieden werden.

In dem vorliegenden Ausführungsbeispiel gemäß Fig. 1 ist die an die Brustprothese 10 angrenzende Oberfläche 142 der Streifen gekrümmt. Daraus ergibt sich zum einen der Vorteil einer leichteren Herstellbarkeit, zum anderen werden ungünstige Krafteinleitungen durch Ecken und Kanten vermieden.

Weisen die Tragestreifen 20 auf ihrer der Brustprothese 10 zugewandten Seite 202 abgerundete Kanten 222 auf, führt dies dazu, daß auch bei ungünstigen Belastungen eine Beschädigung des zweiten elastischen Materials 14 durch die Kanten 222 des Tragestreifens 20 vermieden wird.

Fig. 2 zeigt eine erfindungsgemäße Zwei-Komponenten-Brustprothese 10, deren grundlegender Aufbau auf der Verwendung des ersten 12 und des zweiten elastischen Materials 14 basiert. Erfindungsgemäß erstreckt sich das die Brustprothese 10 aufbauende zweite elastische Material 14 in einen der Randbereiche 22 des Tragestreifens 20 und bewirkt somit in diesem Bereich die elastische Abstützung des Tragestreifens. Im Bereich des anderen Randbereiches 22 des Tragestreifens 20 befindet sich anstelle des in Fig. 1 dargestellten streifenförmig ausgebildeten elastischen Materials 14 eine Kammer 16. Die Schaffung der Kammer 16 hat den Vorteil, daß zum einen die vorteilhaften Wirkungen der erfindungsgemäßen Anordnung des Tragstreifens 20 gewährleistet sind und gleichzeitig eine Vereinfachung der Herstellung dadurch bewirkt wird, daß anstelle des Materials 14 auf einer Seite des Tragstreifens 20 lediglich eine Kammer 16 vorzusehen ist.

Die Verwendung der Zwei-Komponenten-Brustprothese 10 bringt den Vorteil mit sich, daß die günstigen auf der Mehrkomponentenbauweise basierenden Eigenschaften der Brustprothese 10 mit der erfindungsgemäßen Befestigung der Brustprothese 10 mittels eines in den Randbereichen 22 abstützbaren Tragestreifens 20 kombiniert werden können.

Wie in Fig. 1 dargestellt, ergeben sich zwischen der Brustprothese 10 und dem Tragestreifen 20 aufgrund der über die gesamte Breite des Tragestreifens 20 erfolgten Befestigung keinerlei Spalte oder Aussparungen, wodurch ein Reinigen der Brustprothese 10 auf einfache Weise möglich ist.

Durch die Fixierung des Tragestreifens 20 über seine gesamte oder den überwiegenden Teil seiner Breite wird außerdem verhindert, daß beim Abnehmen der Brustprothese 10 die Randbereiche 22 des Tragestreifens 20 in Berührung kommen können und somit verkleben. Auf diese Weise ist eine dauerhafte Benutzung der erfindungsgemäßen Brustprothese 10 möglich.

## Patentansprüche

1. Brustprothese (10), die ein erstes elastisches Material (12), vorzugsweise eine weichelastisch eingestellte additionsvemetzende Zwei-Komponenten-Silikon-Kautschuk-Masse, ein zweites elastischeß Material (14) au der Protheseninnenseite, dessen Elastizität über der des ersten elastischen Materials (12) liegt, sowie einen mit der Brustprothese (10) verbindbaren Tragestreifen (20) zum Zwecke der Befestigung der Brustprothese (10) an der Brust der Trägerin umfaßt,
**dadurch gekennzeichnet,**
**daß** der Tragestreifen (20) der Brustprothese (10) derart angeordnet ist, daß sich sein äußerer und innerer Randbereich (22) jeweils in den Bereich des zweiten elastischen Materials (14) erstreckt, und der mittlere Bereich des Tragestreifens (20) am ersten elastischen Material (12) zu befestigen ist.

2. Brustprothese (10) nach Anspruch 1, **dadurch gekennzeichnet, daß** das zweite elastische Material (14) in Form zweier Streifen ausgeführt ist, die sich auf beiden Randbereichen (22) des Tragestreifens (20) erstrecken.

3. Brustprothese (10) nach Anspruch 2, **dadurch gekennzeichnet, daß** eine oder mehrere der an die Brustprothese (10) angrenzenden Seiten (142) der Streifen eine gekrümmte Oberfläche aufweisen.

4. Brustprothese (10) nach Anspruch 1, **dadurch gekennzeichnet, daß** sich das zweite elastische Material (14) von einem oder beiden der Randbereiche (22) des Tragestreifens (20) in weitere Bereiche der Brustprothese (10) erstreckt.

5. Brustprothese (10) nach einem oder mehreren der Ansprüche 1 bis 4, **dadurch gekennzeichnet, daß** der Tragestreifen (20) in einem Randbereich der Brustprothese (10) angeordnet ist.

6. Brustprothese (10) nach einem oder mehreren der Ansprüche 1 bis 5, **dadurch gekennzeichnet, daß** der Tragestreifen (20) von der Brutprothese (10) lösbar ausgeführt ist.

7. Brustprothese (10) nach einem oder mehreren der Ansprüche 1 bis 6, **dadurch gekennzeichnet, daß** der Tragestreifen (20) mit einer dauernd klebrigen Schicht versehen ist.

8. Brustprothese (10) nach einem oder mehreren der Ansprüche 1 bis 7, **dadurch gekennzeichnet, daß** der Tragestreifen (20) in voneinander beabstandeten Bereichen mit der Brustprothese (10) verbunden ist.

9. Brustprothese (10) nach einem oder mehreren der Ansprüche 1 bis 8, **dadurch gekennzeichnet, daß** der Tragestreifen (20) auf seiner zu der Brustprothese (10) zugewandten Seite (202) abgerundete Kanten (222) aufweist.

## Claims

1. Mammary prosthesis (10), which comprises a first elastic material (12), preferably an addition-crosslinking two-component silicone rubber composition with flexible elastic properties, a second elastic material (14) which is arranged on the inner side of the prosthesis and whose elasticity is greater than that of the first elastic material (12), and a support strip (20) which can be connected to the mammary prosthesis (10) for the purpose of securing the mammary prosthesis (10) on the user's breast, **characterized in that** the support strip (20) of the mammary prosthesis (10) is arranged in such a way that its outer and inner edge area (22) in each case extends into the area of the second elastic material (14), and the central area of the support strip (20) is to be secured on the first elastic material (12).

2. Mammary prosthesis (10) according to Claim 1, **characterized in that** the second elastic material (14) is configured in the form of two strips which extend onto both edge areas (22) of the support strip (20).

3. Mammary prosthesis (10) according to Claim 2, **characterized in that** one or more of the sides (142) of the strips adjacent to the mammary prosthesis (10) have a curved surface.

4. Mammary prosthesis (10) according to Claim 1, **characterized in that** the second elastic material (14) extends from one or both of the edge areas (22) of the support strip (20) into further areas of the mammary prosthesis (10).

5. Mammary prosthesis (10) according to one or more of Claims 1 to 4, **characterized in that** the support strip (20) is arranged in an edge area of the mammary prosthesis (10).

6. Mammary prosthesis (10) according to one or more of Claims 1 to 5, **characterized in that** the support strip (20) is designed to be releasable from the mammary prosthesis (10).

7. Mammary prosthesis (10) according to one or more of Claims 1 to 6, **characterized in that** the support strip (20) is provided with a permanently adhesive layer.

8. Mammary prosthesis (10) according to one or more of Claims 1 to 7, **characterized in that** the support strip (20) is connected to the mammary prosthesis (10) in areas spaced apart from one another.

9. Mammary prosthesis (10) according to one or more of Claims 1 to 8, **characterized in that** the support strip (20) has rounded edges (222) on its side (202) directed towards the mammary prosthesis (10).

## Revendications

1. Prothèse mammaire (10), qui comprend un premier matériau élastique (12), de préférence, une masse de caoutchouc au silicone à deux composants, réglée pour avoir une élasticité molle, réticulant par addition, un deuxième matériau élastique (14) au côté intérieur de la prothèse dont l'élasticité est supérieure à celle du premier matériau élastique (12), ainsi qu'une bande porteuse (20) pouvant être reliée à la prothèse mammaire (10), dans le but de la fixation de la prothèse mammaire (10) au sein de la personne qui la porte,
**caractérisée**
**en ce que** la bande porteuse (20) de la prothèse mammaire (10) est disposée de façon que sa zone de bord extérieure et intérieure (22) s'étend respectivement dans la zone du deuxième matériau élastique (14), et que la zone médiane de la bande porteuse (20) doit être fixée au premier matériau élastique (12).

2. Prothèse mammaire (10) selon la revendication 1, **caractérisée en ce que** le deuxième matériau élastique (14) est réalisé sous forme de deux bandes qui s'étendent sur les deux zones de bord (22) de la bande porteuse (20).

3. Prothèse mammaire (10) selon la revendication 2, **caractérisée en ce qu'**un ou plusieurs des côtés (142) des bandes avoisinant la prothèse mammaire (10) présentent une surface courbée.

4. Prothèse mammaire (10) selon la revendication 1, **caractérisée en ce que** le deuxième matériau élastique (14) s'étend depuis l'une ou les deux des zones de bord (22) de la bande porteuse (20) dans d'autres zones de la prothèse mammaire (10).

5. Prothèse mammaire (10) selon l'une ou plusieurs des revendications 1 à 4, **caractérisée en ce que** la bande porteuse (20) est disposée dans une zone de bord de la prothèse mammaire (10).

6. Prothèse mammaire (10) selon l'une ou plusieurs des revendications 1 à 5, **caractérisée en ce que** la bande porteuse (20) est réalisée pour pouvoir être détachée de la prothèse mammaire (10).

7. Prothèse mammaire (10) selon l'une ou plusieurs des revendications 1 à 6, **caractérisée en ce que** la bande porteuse (20) est pourvue d'une couche collante permanente.

8. Prothèse mammaire (10) selon l'une ou plusieurs des revendications 1 à 7, **caractérisée en ce que** la bande porteuse (20) est reliée à des zones espacées les unes des autres à la prothèse mammaire (10).

9. Prothèse mammaire (10) selon l'une ou plusieurs des revendications 1 à 8, **caractérisée en ce que** la bande porteuse (20) présente des arêtes arrondies (222) sur son côté (202) orienté vers la prothèse mammaire (10).
